# EUROPÄISCHE PATENTANMELDUNG

(11) **EP 0 864 566 A2**
(43) Veröffentlichungstag der Anmeldung: **16.09.1998**
(21) Anmeldenummer: 98103234.5
(22) Anmeldetag: 25.02.1998
(51) Int. Cl.: C07D 241/46, C07C 39/14, C07C 215/86

(54) **Verfahren zur Herstellung von Hydroxynaphthalinen**

(30) Priorität: 10.03.1997 DE 19709701
(71) Anmelder: BAYER AG, 51368 Leverkusen (DE)
(72) Erfinder: Pelster, Thomas, Dr., 50999 Köln (DE)

(57) **Zusammenfassung**

Es wurde ein Verfahren zur Herstellung von Hydroxynaphthalinen der Formel (I) 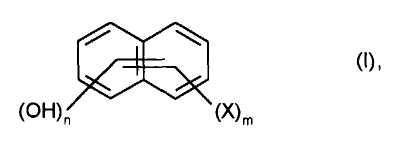 worin
- n: für eine Zahl von 1 bis 6, vorzugsweise 1 oder 2 steht,
- X: C₁-C₆-Alkyl, Halogen, Carboxyl, Carbonsäureester, Nitro, Chlorsulfonyl, Arylsulfonyl, Hydroxy, Alkoxy, Acyloxy oder ein gegebenenfalls durch Alkyl oder Aryl substituiertes Amino oder Aminosulfonyl, bedeutet, oder zwei Reste X zusammen mit den benachbarten C-Atomen, an die sie gebunden sind, einen ankondensierten aromatischen, cycloaliphatischen oder heterocyclischen Ring bilden,
- m: eine Zahl von 0 bis 6 bedeutet, wobei für
- m: größer als 1,
- X: jeweils verschiedene oder gleiche der oben genannten Bedeutungen haben kann,
dadurch gekennzeichnet, daß man Naphthalinsulfonsäuren der Formel (II) oder deren Salze 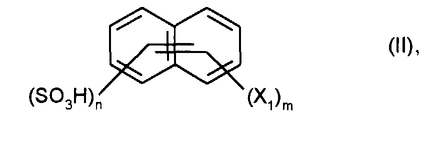 wobei n und m die oben angegebene Bedeutung haben und
- X₁: die gleiche Bedeutung wie X hat, jedoch gegebenenfalls anstelle von an aromatischen Resten gebundenen OH-Substituenten SO₃H-Substituenten stehen,
mit Alkali in Gegenwart eines alkylierten Harnstoffderivats umsetzt, gefunden, wobei die so hergestellten Hydroxynaphthaline sich beispielsweise zum Massefärben von Kunststoffen sowie als Vorprodukte zur Herstellung von Diisocyanaten, Pigmenten oder Farbstoffen eignen.

## Beschreibung

Die Erfindung betrifft ein Verfahren zur Herstellung von Hydroxynaphthalinen sowie die Verwendung dieser Verfahrensprodukte zum Massefärben von Kunststoffen oder als Vorprodukte, beispielsweise zur Herstellung von Diisocyanaten, Pigmenten oder Farbstoffen.

Hydroxynaphthaline wie beispielsweise das 8,8'-Dihydroxynaphthazin sind gemäß DE-A 21 48 850 zum Massefärben von Kunststoffen beschrieben. Hergestellt werden diese Verbindungen gemäß dem in DE-A 39 33 932 offenbarten Weg ausgehend von der entsprechenden Disulfonsäureverbindung in Gegenwart eines wasserhaltigen alkoholisch-alkalischen Mediums. Nachteilig bei dieser Verfahrensvariante ist beispielsweise eine noch zu verbessernde Raum-Zeit-Ausbeute. Außerdem ist die Übertragbarkeit auf den technischen Maßstab problematisch, da aufgrund der teilweisen H₂-Entwicklung unter den jeweiligen Reaktionsbedingungen durch Zersetzung des verwendeten Lösungsmittels aufwendige Sicherheitsvorkehrungen vorgenommen werden müßten.

Es wurde nun ein Verfahren zur Herstellung von Hydroxynaphthalinen der Formel (I) gefunden, 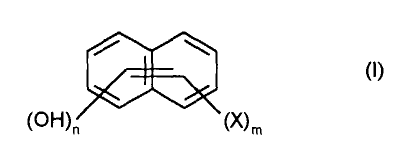 worin
- n: für eine Zahl von 1 bis 6, vorzugsweise 1 oder 2 steht,
- X: C₁-C₆-Alkyl, Halogen, Carboxyl, Carbonsäureester, Nitro, Chlorsulfonyl, Arylsulfonyl, Hydroxy, Alkoxy, Acyloxy oder ein gegebenenfalls durch Alkyl oder Aryl substituiertes Amino oder Aminosulfonyl, bedeutet, oder zwei Reste X zusammen mit den benachbarten C-Atomen, an die sie gebunden sind, einen ankondensierten aromatischen, cycloaliphatischen oder heterocyclischen Ring bilden,
- m: eine Zahl von 0 bis 6 bedeutet, wobei für
- m: größer als 1,
- X: jeweils verschiedene oder gleiche der oben genannten Bedeutungen haben kann,
dadurch gekennzeichnet, daß man Naphthalinsulfonsäuren der Formel (II) oder deren Salze, vorzugsweise deren Alkalisalze wie Na- oder K-Salze, 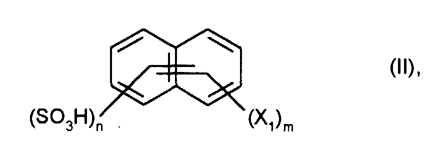 wobei n und m die oben angegebene Bedeutung haben und
- X₁: die gleiche Bedeutung wie X hat, jedoch gegebenenfalls anstelle von an aromatischen Resten gebundenen OH-Substituenten SO₃H-Substituenten stehen,
mit Alkali in Gegenwart eines alkylierten Harnstoffderivats umsetzt.

Mit der gegebenenfalls anderen Bedeutung von X₁ gegenüber X wird berücksichtigt, daß nicht nur die Sulfogruppen des Naphthalinkerns, sondern auch die an anderen aromatischen Resten des Ausgangsprodukts in OH-Substituenten überführt werden.

Das erfindungsgemäße Verfahren eignet sich besonders zur Herstellung von Verbindungen der Formel (I), worin
- m: für eine Zahl von 2 bis 6, insbesondere für 2 oder 3 steht und zwei Reste
- X: zusammen einen Rest der Formel (a) 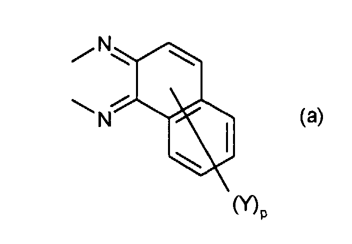 oder (b) 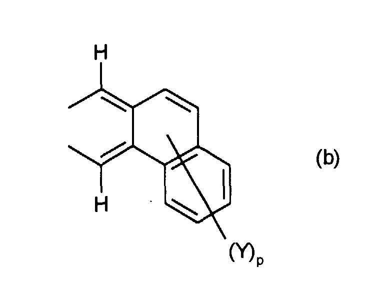
bedeuten, der an zwei benachbarte C-Atome des Naphthalinringes verknüpft ist,
worin
- Y: C₁-C₆-Alkyl, Halogen, insbesondere Cl, COOH, Carbonsäureester, insbesondere C₁-C₄-Alkylester, Nitro, Chlorsulfonyl, Arylsulfonyl, Hydroxy, Alkoxy, Acyloxy oder ein gegebenenfalls durch Alkyl oder Aryl substituiertes Amino oder Aminosulfonyl bedeutet,
- p: eine Zahl von 0 bis 6 bedeutet, wobei für
- p: größer als 1,
- Y: jeweils verschiedene oder gleiche der oben genannten Bedeutungen haben kann.

Verbindungen der Formel I, worin zwei Reste X₁ zusammen für einen Rest der Formel (a) stehen, werden im folgenden als Verbindungen der Formel (Ia) bezeichnet. Verbindungen der Formel I, worin zwei Reste X zusammen einen Rest der Formel (b) bedeuten, werden im folgenden als Verbindungen (Ib) bezeichnet.

In einer besonderen Ausführungsform ist der Rest a) oder b) in 1,2-Stellung, des Naphthalinringes angebunden.

Besonders bevorzugt sind Verbindungen der Formel (I), die der Formel (Ic) entsprechen 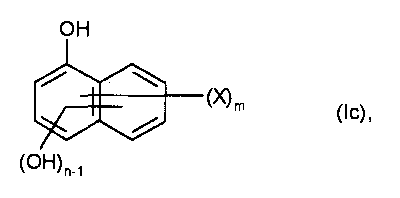 worin
- n: für 1 oder 2 steht, und X und m die oben genannte Bedeutung besitzen.

Im Rahmen dieser Anmeldung und sofern nichts anderes beschrieben ist, steht vorzugsweise Alkyl für C₁-C₄-Alkyl, Aryl für Phenyl und Alkoxy für C₁-C₄-Alkoxy, wobei diese Reste gegebenenfalls substituiert sind.

Insbesondere sind dies die nachfolgend genannten Verbindungen

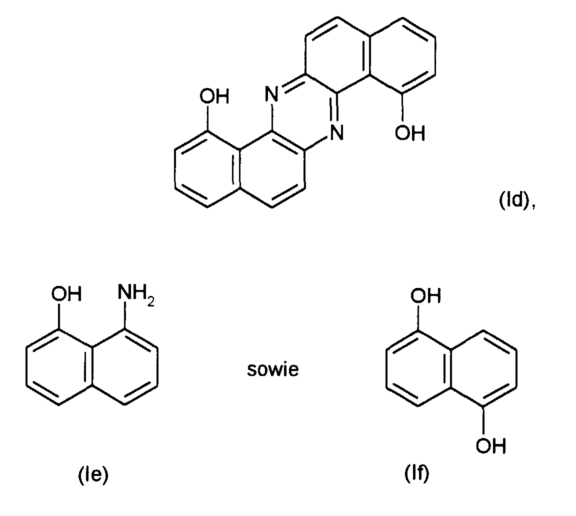

Als alkylierte Harnstoffderivate kommen beispielsweise offenkettige in Frage wie Dialkylharnstoffe, insbesondere Di-C₁-C₄-Alkylharnstoffe wie Dimethylharnstoff oder Tetraalkylharnstoffe, insbesondere Tetra-C₁-C₄-alkylharnstoffe wie Tetramethylharnstoff oder cyclische Harnstoffderivate wie N,N'-Dialkylalkylenharnstoffe, insbesondere N,N'-Di-C₁-C₄-alkyl-C₂-C₄-alkylenharnstoffe, besonders bevorzugt N,N'-Di-methylethylenharnstoff der Formel 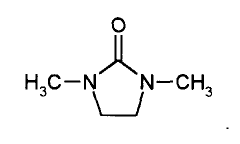 sowie N,N'-Dimethylpropylenharnstoff der Formel 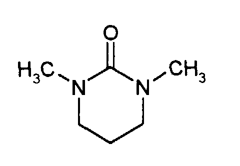 oder N,N',N''-Trialkylhexahydro-1,3,6-triazin-2-one, wie 1,3,6-Trimethylhexahydro-1,3,6-triazin-2-on der Formel

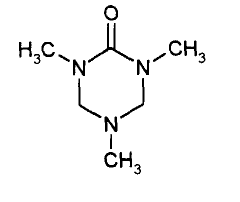

Derartige cyclische Harnstoffderivate sind beispielsweise aus EP-A 688 829 bzw. aus DE-A 42 17 954 bekannt.

Das alkylierte Harnstoffderivat oder Mischung alkylierter Harnstoffderivate wird vorzugsweise in einer Menge von 0,005 bis 10 Gew.-Teile, bevorzugt 0,1 bis 5 Gew.-Teile, besonders bevorzugt 1 bis 3 Gew.-Teilen pro Gew.-Teil der Naphthalinsulfonsäureverbindung der Formel (II) eingesetzt.

Gegebenenfalls wird das alkylierte Harnstoffderivat in einer Mischung mit einem inerten organischen Lösungsmittel wie Dimethylsulfoxid (DMSO), Pyridin, Toluol, Xylol oder Nitrobenzol eingesetzt.

Im Rahmen dieser Anmeldung werden unter Alkali sowohl die Hydroxide der Alkalimetalle sowie die der Erdalkalimetalle verstanden. Geeignete Alkalien sind Alkalihydroxide, insbesondere NaOH oder KOH sowie Ca(OH)₂. Bevorzugt werden 1 bis 3 Gew.-Teile Alkali pro 1 Gew.-Teile der Naphthalinsulfonsäure der Formel (II) eingesetzt, insbesondere 1 bis 2 Gew.-Teile.

Das erfindungsgemäße Verfahren kann auch in Gegenwart von Wasser durchgeführt werden, wobei vorzugsweise nicht mehr als 10 Gew.-%, bezogen auf das Reaktionsmedium an Wasser eingesetzt wird. Unter Reaktionsmedium werden in der Regel alle Inhaltsstoffe außer den Verbindungen der Formeln (I) und (II) und Alkali verstanden werden. In einer bevorzugten Ausführungsform enthält das Reaktionsmedium 40 bis 100 % des alkylierten Harnstoffderivats, 0 bis 10 % Wasser und 0 bis 10 % eines inerten organischen Lösungsmittels, wobei sich die Summe aller Bestandteile zu 100 % ergibt.

Das erfindungsgemäße Verfahren wird im allgemeinen bei einer Temperatur von wenigstens 120°C durchgeführt, bevorzugt bei 150 bis 180°C.

Sofern im Reaktionsmedium Wasser oder andere unterhalb der Reaktionstemperatur siedende Verbindungen enthalten sind, wird die Umsetzung vorzugsweise unter Druck durchgeführt. Das erfindungsgemäße Verfahren erfolgt dann beispielsweise im Autoklaven, wobei ein Druck von 1 bis 5 bar bevorzugt ist.

Nach beendeter Reaktion, die chromatographisch verfolgt werden kann, wird die Reaktionsmischung vorzugsweise auf eine Temperatur von 50-70°C abgekühlt und mit einem Alkohol, beispielsweise Methanol, mit Pyridin, Pyridin-Wasser-Gemischen oder direkt mit Wasser verdünnt und das Reaktionsprodukt in üblicher Weise durch Abfiltrieren und Waschen isoliert.

Die Erfindung betrifft auch ein Verfahren zum Massefärben von Kunststoffen unter Verwendung der nach dem erfindungsgemäßen Verfahren erhaltenen Farbstoffen, vorzugsweise solcher der Formel Ia oder Ib sowie die damit gefärbten Kunststoffe.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei dem der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester und Polyamide.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, u.a.

Weiterhin geeignete Polyester sind: Polyethylenterephthalate, Polycarbonate und Celluloseester.

Bevorzugt sind Polystyrol, Styrol-Mischpolymere, Polycarbonate und Polymethacrylat. Besonders bevorzugt ist Polystyrol.

Die erwähnten hochmolekularen Verbindungen können einzeln oder in Gemischen, als plastische Massen oder Schmelzen vorliegen.

Die erfindungsgemäß erhaltenen Farbstoffe der Formel Ia oder Ib werden vorzugsweise in feinverteilter Form zur Anwendung gebracht, wobei Dispergiermittel mitverwendet werden können, aber nicht müssen.

Werden die Farbstoffe Ia oder Ib nach der Polymerisation eingesetzt, so werden sie mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguß-Verfahren zu Formteilen weiterverarbeitet.

Da die Farbstoffe der Formel Ia oder Ib gegenüber Polymerisationskatalysatoren, insbesondere Peroxiden, beständig sind, ist es auch möglich, die Farbstoffe den monomeren Ausgangsmaterialien zuzusetzen und dann in Gegenwart von Polymerisationskatalysatoren zu polymerisieren. Dazu werden die Farbstoffe vorzugsweise in den monomeren Komponenten gelöst oder mit ihnen innig vermischt.

Die Farbstoffe der Formel Ia oder Ib werden vorzugsweise zum Färben der genannten Polymeren in Mengen von 0,0001 bis 1 Gew.-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Nach dem erfindungsgemäßen Verfahren erhält man transparente bzw. gedeckte brillante Färbungen mit guter Hitzbeständigkeit sowie guter Licht- und Wetterechtheit.

In das erfindungsgemäße Färbeverfahren können auch Mischungen verschiedener Farbstoffe, vorzugsweise Farbstoffe der Formel Ia und/oder Ib und/oder Mischungen von Farbstoffen der Formel Ia oder Ib mit anderen Farbstoffen und/oder anorganischen bzw. organischen Pigmenten eingesetzt werden.

Die Erfindung betrifft weiterhin die Verwendung der nach dem erfindungsgemäßen Verfahren erhaltenen Verbindungen der Formel (I), insbesondere solche der Formel Ic als Vorprodukte zur Herstellung von Isocyanaten sowie Pigmenten.

Die Herstellung von Isocyanaten erfolgt dabei beispielsweise durch Umsetzung von Dihydroxynaphthalin der Formel (If) mit Ammoniak/Bisulfit (Bucherer Reaktion) und anschließend mit Phosgen.

Die Herstellung von Farbstoffen z.B. Azofarbstoffen erfolgt dabei beispielsweise durch Diazotierung eines aromatischen Amins und Kupplung auf Verbindungen der Formel I. Als aromatisches Amin kann beispielsweise 2-Aminophenol-4-sulfonsäure und als Verbindung der Formel I das Dihydroxynaphthalin der Formel (If) genannt werden.

### Beispiele

### Beispiel 1

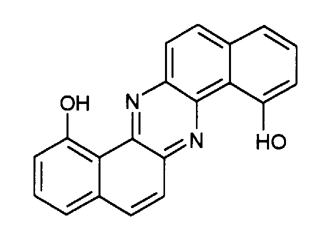

Eine Mischung aus 100 g N,N'-Dimethylethylenharnstoff und 60 g Kaliumhydroxid wurde auf 130°C erhitzt. In diese Mischung wurden unter Stickstoffatmosphäre innerhalb von 30 Minuten 22 g (0,5 mol) 8,8'-Naphthazindisulfonsäure eingetragen. Das Reaktionsgemisch wurde 18 Stunden lang auf 160°C erhitzt, bis chromatographisch kein Ausgangsprodukt mehr nachweisbar war. Anschließend wurde auf 80°C abgekühlt, mit 400 ml Wasser versetzt, das Produkt abfiltriert, mit Wasser neutral gewaschen und getrocknet. Man erhielt 14 g (92 % d.Th.) 8,8'-Dihydroxynaphthazin, das in dieser Form Kunststoffe in klaren gelben Nuancen färbt.

### Beispiel 2

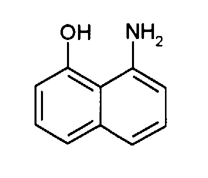

Eine Mischung aus 100 g N,N'-Dimethylpropylenharnstoff und 60 g Kaliumhydroxid wurde auf 150°C erhitzt. In diese Mischung wurden unter Stickstoffatmosphäre innerhalb von 30 Minuten 22 g 1-Amino-naphthazin-8-sulfonsäure eingetragen. Das Reaktionsgemisch wurde 12 Stunden auf 160°C erhitzt, bis chromatographisch kein Ausgangsprodukt mehr nachweisbar war. Anschließend wurde auf 80°C abgekühlt, mit 400 ml Wasser versetzt, mit Salzsäure neutralisiert, das Produkt abfiltriert, mit Wasser gewaschen und getrocknet. Man erhielt 12 g (75 % d.Th.) 1-Amino-8-hydroxynaphthalin.

### Beispiel 3

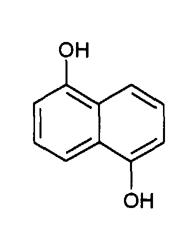

Eine Mischung aus 100 g N,N'-Dimethylpropylenharnstoff und 60 g Kaliumhydroxid wurde auf 150°C erhitzt. In diese Mischung wurden unter Stickstoffatmosphäre innerhalb von 30 Minuten 29 g (0,1 mol) 1,5-Naphthalindisulfonsäure eingetragen. Das Reaktionsgemisch wurde 22 Stunden auf 160°C erhitzt, bis chromatographisch kein Ausgangsprodukt mehr nachweisbar war. Anschließend wurde auf 80°C abgekühlt, mit 400 ml Wasser versetzt, mit Salzsäure neutralisiert, das Produkt abfiltriert, mit Wasser gewaschen und getrocknet. Man erhielt 14 g (87 % d.Th.) 1,8-Dihydroxynaphthalin, das in der vorliegenden Form als Farbstoffzwischenprodukt und zur Herstellung von Isocyanaten verwendet werden kann.

## Patentansprüche

1. Verfahren zur Herstellung von Hydroxynaphthalinen der Formel (I) 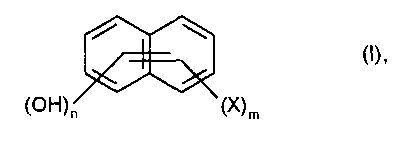 worin
n für eine Zahl von 1 bis 6, vorzugsweise 1 oder 2 steht,
X C₁-C₆-Alkyl, Halogen, Carboxyl, Carbonsäureester, Nitro, Chlorsulfonyl, Arylsulfonyl, Hydroxy, Alkoxy, Acyloxy oder ein gegebenenfalls durch Alkyl oder Aryl substituiertes Amino oder Aminosulfonyl, bedeutet, oder zwei Reste X zusammen mit den benachbarten C-Atomen, an die sie gebunden sind, einen ankondensierten aromatischen, cycloaliphatischen oder heterocyclischen Ring bilden,
m eine Zahl von 0 bis 6 bedeutet, wobei für
m größer als 1,
X jeweils verschiedene oder gleiche der oben genannten Bedeutungen haben kann,
dadurch gekennzeichnet, daß man Naphthalinsulfonsäuren der Formel (II) oder deren Salze 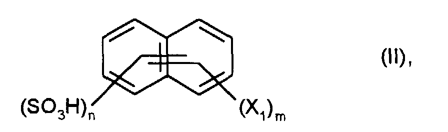 wobei n und m die oben angegebene Bedeutung haben und
X₁ die gleiche Bedeutung wie X hat, jedoch gegebenenfalls anstelle von an aromatischen Resten gebundenen OH-Substituenten SO₃H-Substituenten stehen,
mit Alkali in Gegenwart eines alkylierten Harnstoffderivats umsetzt.

2. Verfahren gemäß Anspruch 1, dadurch gekennzeichnet, daß
m für eine Zahl von 2 bis 6, insbesondere für 2 oder 3 steht und zwei Reste
X zusammen einen Rest der Formel 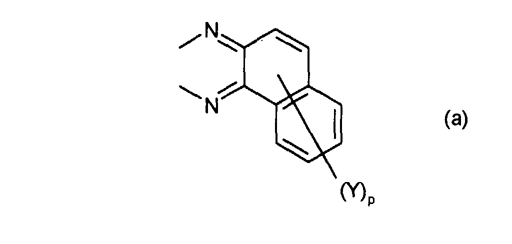 oder 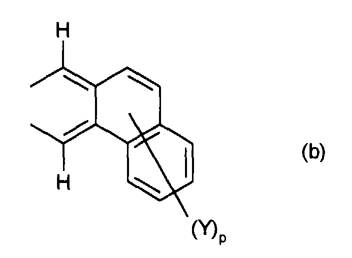
bedeuten, der an zwei benachbarte C-Atome des Naphthalinringes geknüpft ist,
worin
Y C₁-C₆-Alkyl, Halogen, insbesondere Cl, COOH, Carbonsäureester, Nitro, Chlorsulfonyl, Arylsulfonyl, Hydroxy, Alkoxy, Acyloxy oder ein gegebenenfalls durch Alkyl oder Aryl substituiertes Amino oder Aminosulfonyl bedeutet,
p eine Zahl von 0 bis 6 bedeutet, wobei für
p größer als 1,
Y jeweils verschiedene oder gleiche der oben genannten Bedeutungen haben kann.

3. Verfahren gemäß Anspruch 1, dadurch gekennzeichnet, daß die Verbindungen der Formel (I) der Formel (Ic) entsprechen 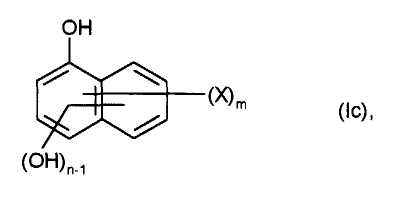 worin
n für 1 oder 2 steht.

4. Verfahren gemäß Anspruch 1, dadurch gekennzeichnet, daß als Verbindungen der Formel (I) eine der nachstehenden Verbindungen hergestellt wird

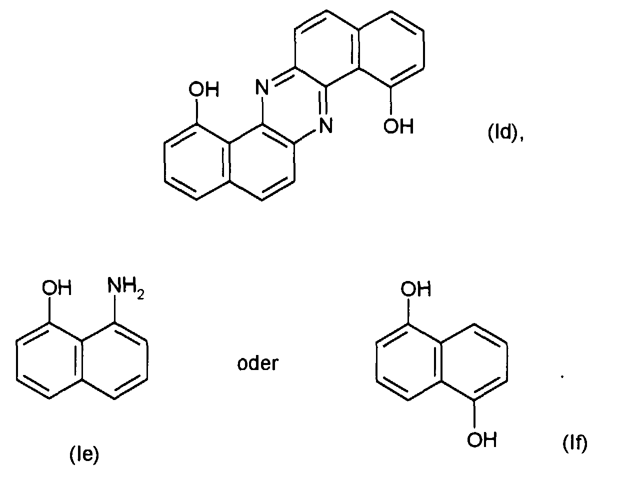

5. Verfahren gemäß Anspruch 1, dadurch gekennzeichnet, daß als alkylierte Harnstoffderivate offenkettige oder cyclische Harnstoffderivate verwendet werden.

6. Verfahren gemäß Anspruch 1, dadurch gekennzeichnet, daß als alkylierte Harnstoffderivate Dialkylharnstoffe, insbesondere Di-C₁-C₄-Alkylharnstoffe wie Dimethylharnstoff oder Tetraalkylharnstoffe, insbesondere Tetra-C₁-C₄-alkylharnstoffe wie Tetramethylharnstoff, N,N'-Dialkylalkylenharnstoffe oder N,N',N''-Trialkylhexahydro-1,3,6-triazin-2-on eingesetzt werden.

7. Verfahren gemäß Anspruch 1, dadurch gekennzeichnet, daß als alkyliertes Harnstoffderivat N,N'-Dimethylethylenharnstoff der Formel 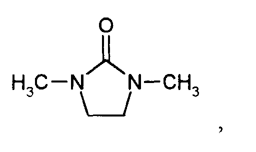 N,N'-Dimethylpropylenharnstoff der Formel 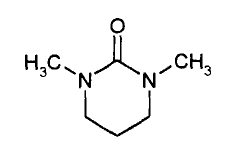 oder 1,3,6-Trimethylhexahydro-1,3,6-triazin-2-on der Formel 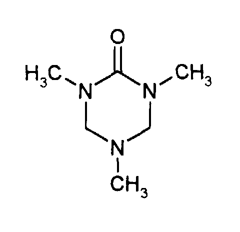 eingesetzt wird.

8. Verfahren gemäß Anspruch 1, dadurch gekennzeichnet, daß man die Umsetzung bei einer Temperatur von wenigstens 120°C durchführt.

9. Verfahren zum Massefärben von Kunststoffen, dadurch gekennzeichnet, daß man nach dem Verfahren gemäß Anspruch 1 erhaltenen Farbstoffe, insbesondere die nach dem Verfahren gemäß Anspruch 2 erhaltenen Farbstoffe, verwendet.

10. Kunststoffe gefärbt nach dem Verfahren gemäß Anspruch 9.
